# EUROPEAN PATENT APPLICATION

(11) **EP 1 033 240 A1**
(43) Date of publication of application: **06.09.2000**
(21) Application number: 00301311.7
(22) Date of filing: 21.02.2000
(51) Int. Cl.: B31F 1/07, A61F 13/15

(54) **Method and apparatus for embossing sheet materials such as paper webs and diaper backsheets**

(30) Priority: 14.05.1999 US 312531; 01.03.1999 US 122120 P
(71) Applicant: Industrie Cartarie Tronchetti S.p.A., 55023 Borgo a Mozzano (Lucca) (IT)
(72) Inventor: Tronchetti, Sauro, 55100-Lucca (IT)
(74) Representative: Neill, Alastair William

(57) **Abstract**

A method of manufacturing a diaper, the method including: providing a sheet of diaper backsheet material (18); embossing the backsheet material (18) by compressing the backsheet material (18) between a hard roller (1) and a resilient roller (10); and combining the embossed backsheet material (18) with other materials to provide a finished diaper.

## Description

### FIELD OF THE INVENTION

The present invention relates to a method for forming rollers, particularly rollers for embossing paper webs for tissue products, such as for example kitchen paper, toilet paper, et cetera. The invention also relates to methods and apparatus for embossing materials, such as tissue paper and diaper backsheets.

### BACKGROUND

Embossing rollers that are known in the art are made by various methods. For example, one prior art method for making an embossing roller includes forming a single rib on a circumferential surface of a roller and then removing portions of the rib to form a pattern.

Another prior art method includes a complex chemical and mechanical process for making a roller, which includes covering a copper roll with asphaltum and then knurling the asphaltum to form therein grooves separated by ridges of asphaltum. The ridges extend across the entire width of the roll in the cross machine direction. The entire roll is then coated with a mercurous nitrate coating, the mercurous nitrate is converted to mercurous chloride, and then a design is outlined by removing both the mercurous chloride coating and the asphaltum so that the design is outlined by bare copper. The mercurous chloride coating inside the design is then removed and both the mercurous chloride coating and the asphaltum outside the design are removed, leaving the desired design defined by asphaltum ridges separated by "ground lines" or grooves. In this prior art method, the design is ultimately formed by the asphaltum left behind on the roller, and the design is surrounded by bare roll where the asphaltum has been removed.

Another prior art method for making an embossing roller includes etching a roller with a master tool to form the roller in a single step.

U.S. Patent No. 5,147,347 discloses a prior art diaper. This patent discloses at column 6, lines 20-22, "In the shown embodiment, outer cover 12 is also embossed or otherwise textured to provide a non-glossy, matte finish."

### DESCRIPTION OF THE PRIOR ART

Paper embossing machines, for example for producing composite two-ply kitchen paper rolls, include two embossing rollers: each roller has a series of protrusions arranged in parallel rows and cooperates with a respective presser roller to emboss each sheet.

The embossing rollers are placed close together, so as to join the two embossed sheets, preferably by making the points coincide. One of the two embossing rollers is coated with glue to paste the two sheets together.

The protrusions of the embossing rollers are provided by machining, or by means of chemical etching, so that the sheets have a uniform stippling that is constituted by parallel rows of points directed inward, that is to say, on the mutually facing sides of the sheets.

An advantageous condition for making the two sheets remain stably associated, however, is to have the largest possible number of protrusions of one sheet match those of the other sheet, in order to ensure a wide pasting surface. As an alternative, in the so-called "staggered" system, the protrusions of one sheet must coincide with the spaces between the protrusions of the other sheet.

GB-A-2166690 discloses an embossing process producing embossed paper sheets, such as towels, comprising embossments having a major and minor axis wherein the major axis of substantially all of the embossments is aligned in the cross machine direction.

WO-A-93/25383 discloses a modular pattern roll, for embossing paper, made by assembling separate components rather than being one piece and chemically etched. This construction has advantages but is however complex and expensive.

An aim of the present invention is to provide a method for producing rollers, particularly rollers for embossing paper webs, which are capable to form complex ornamental patterns on the finished product.

An object of the invention is to provide a method that is considerably cheaper, from the production point of view, than conventional systems.

A further object is to provide a method that is capable of producing ornamental patterns or designs with various degrees of complexity, according to the requirements, but always with extreme precision and reproducibility, even mirror-symmetrical reproducibility, on multiple rollers.

### SUMMARY

This aim, these objects, and others which will become apparent hereinafter are achieved by a method for producing rollers, particularly rollers for embossing paper webs, comprising a first step of forming a series of protrusions and a second step of forming a pattern or design which includes the at least partial removal of a certain number of the protrusions.

The quality of the embossed pattern produced by rolls depends upon the method by which the rolls are made and the quantity and nature of the sheet material to be embossed. In the prior art, highly precise embossed patterns on tissue paper could be achieved only in the paper-making stage, as opposed to the converting stage, which adds to the cost of the final product. For other types of sheet materials, such as plastic used for diaper backings, the quality of the imprint may depend on the quality of the embossing roll used, and/or the quality of the non-woven materials used.

An object of the present invention is to provide a method for producing rollers, particularly rollers for embossing sheets or webs of material, such as tissue paper and diaper backsheets, which are capable of forming complex ornamental patterns having a crisp defined contour on the finished product.

Another object of the invention is to provide a method that is considerably cheaper, from the production point of view, than conventional systems.

A further object is to provide a method that is capable of producing ornamental patterns or designs with various degrees of complexity, according to the requirements, but always with extreme precision and reproducibility, even mirror-symmetrical reproducibility, on multiple rollers.

The invention provides a method of manufacturing a diaper, the method comprising the steps of: providing a sheet of diaper backsheet material; embossing the backsheet material by compressing the backsheet material between a hard roller and a resilient roller; and combining the embossed backsheet material with other materials to provide a finished diaper.

This method improves the aesthetics of the backsheet material, improves the hand feel of the material by making the material softer, and makes the material more cloth-like, adding considerable value to the product through differentiation. Preferably, the backsheet material includes relatively low-caliber raw materials having relatively low cost. Either a brand name or graphics can be embossed on the material. The hard roller used in the method is preferably made of steel.

The invention also provides a method for embossing a web of diaper backsheet material, the method comprising the steps of: providing a first embossing roller by forming on the outer surface of a generally cylindrical roller a series of protrusions, and forming on the roller a pattern or design by at least partially removing a certain number of said protrusions so that the remaining protrusions surround the pattern or design; providing a second roller; and compressing the sheet of material between the first and second rollers.

Preferably, the first roller used in the method is made of steel and the second roller is resilient. The method may further comprise a third roller that is made of steel, and a second sheet of material. The resilient roller rotates in one direction to compress the first sheet of material between the steel first roller and the resilient second roller, and the resilient roller rotates in the opposite direction to compress the second sheet of material between the resilient second roller and the steel third roller.

The invention also provides a method for embossing a web of diaper backsheet material, the method comprising the steps of: providing a first embossing roller having a width in the cross machine direction and having a circumference. The first embossing roller is provided by forming on the outer surface of a generally cylindrical roller a series of protrusions, each of the protrusions having a protrusion width in the cross machine direction that is substantially less than the width of the roller. Each of the protrusions also has a protrusion dimension in the direction of the circumference of the roller, the protrusion dimension being substantially less than the circumference of the roller. A pattern or design is formed on the roller by at least partially removing a certain number of the protrusions. The method also includes the steps of providing a second roller, and compressing the sheet of material between the first and second rollers.

The steps of forming and then removing some of the protrusions to form the desired pattern allow greater flexibility in making a roller. In this method, the protrusions can be removed with a device programmed to make various designs across the roller. The method of the present invention therefore provides more precise and creative designs. Furthermore, the method of the present invention is very simple, unlike prior art methods.

Other features and advantages of the invention will become apparent to those skilled in the art upon review of the following detailed description, claims, and drawings.

Further characteristics and advantages will become apparent from the description of a preferred but not exclusive embodiment of the invention, illustrated only by way of nonlimitative example in the accompanying drawings, wherein:

### BRIEF DESCRIPTION OF THE DRAWINGS

Fig. 1 is a schematic perspective view of an embossing roller according to the invention; and
Fig. 2 is an enlarged partial view of a portion of the surface of the roller according to the invention.
Fig. 3 is an end elevational view of a set of rollers embossing a sheet of material according to the method of the present invention.
Fig. 4 is an exploded partial view of a diaper.
Figs. 5-12 illustrate example patterns embossed on a sheet of material.
Fig. 13 is an end elevational view of an alternative roller set.
Fig. 14 is a side elevational view of the resilient roller of Fig. 13.
Fig 15 is an end elevational view of an alternative roller set.
Fig. 16 is an end elevational view of an alternative roller set.

Before one embodiment of the invention is explained in detail, it is to be understood that the invention is not limited in its application to the details of construction and the arrangements of the components set forth in the following description or illustrated in the drawings. The invention is capable of other embodiments and of being practiced or being carried out in various ways. Also, it is understood that the phraseology and terminology used herein is for the purpose of description and should not be regarded as limiting. The use of "including" and "comprising" and variations thereof herein is meant to encompass the items listed thereafter and equivalents thereof as well as additional items. The use of letters to identify steps of a method or process is simply for identification and is not meant to indicate that the steps should be performed in a particular order.

### DESCRIPTION OF A PREFERRED EMBODIMENT OF THE INVENTION

The embossing roller according to the invention, generally designated by the reference numeral 1, is suitable to be used in a substantially conventional machine for producing embossed paper, of the type that includes two embossing rollers mounted with parallel axes, so that the embossing surface of the two rollers is in contact at the points of the facing protrusions of each roller.

In a per se known manner, each embossing roller cooperates with a respective presser roller, made of rubber or other flexible material, to provide embossing on the respective paper web. Two paper webs are in fact fed respectively in the first pair and in the second pair of embossing and presser rollers in order to be embossed. Once embossed, the two webs are joined at the line of contact between the two embossing rollers so as to obtain a two-ply paper web.

According to the invention, the embossing roller is produced with a first step of forming a uniform series of protrusions 3, for example by machining. It is possible to perform optional finishing operations, or one may move on directly to a second step of forming an ornamental pattern or design 5 that includes the at least partial removal of a certain number of protrusions.

The protrusions may be advantageously removed by means of a numeric-control machine, for example a milling machine. This allows to reproduce each ornamental pattern or design perfectly, and even in a symmetrical manner, on the second roller. It is in fact very important to match the highest possible number of facing points of the protrusions of the two rollers, in order to ensure good gluing of the two plies.

Indeed, by providing the design in a symmetrically identical manner on the two embossing rollers, all the points of one roller coincide with all the points of the other roller, ensuring the maximum number of gluing points between the two plies.

It has been observed in practice that the invention achieves the intended aim and objects, a method having been provided for forming embossing rollers with very complex ornamental patterns.

The method according to the invention is susceptible of numerous modifications and variations, all of which are within the scope of the appended claims. All the details may be replaced with technically equivalent elements.

For example, the described steps can also be performed in reverse order.

The materials employed, as well as the dimensions, may of course be any according to the requirements and the state of the art.

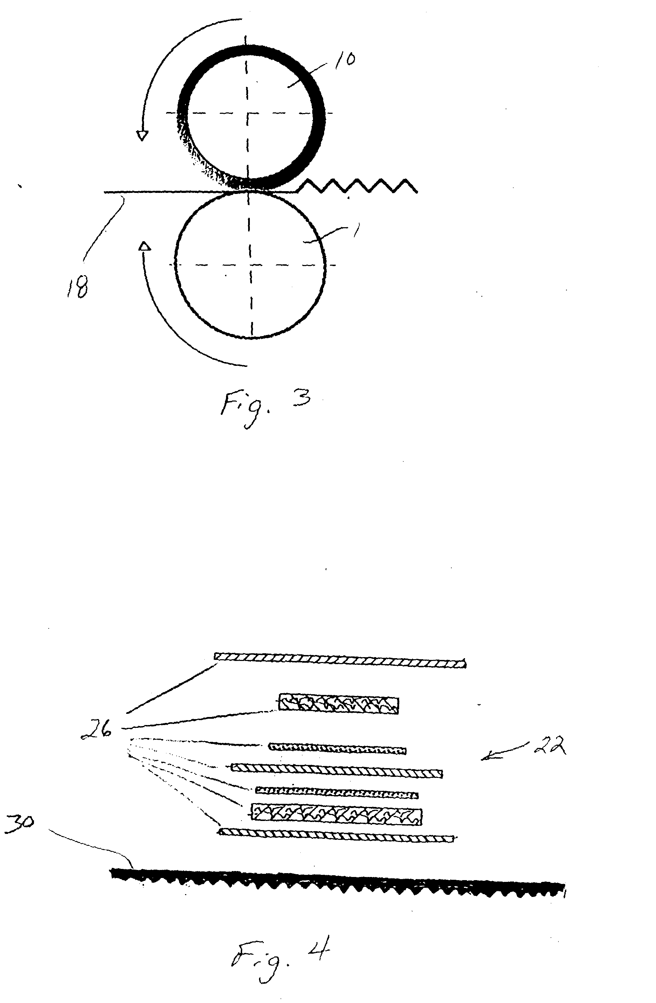

Fig. 3 illustrates a set of rollers including a resilient (e.g., rubber) roller 10 and a hard roller 1 (e.g., an embossing roller). Preferably, the hard roller 1 is made of steel. Except as described below, the hard roller 1 is preferably made according to the methods described in my co-pending U.S. Application No. 08/816,345, filed March 13, 1997 and co-pending U.S. Provisional Application No. 60/122,120, filed March 1, 1999, both of which are hereby incorporated by reference.

The rollers 10, 1 may be incorporated into a machine for embossing a sheet or web of material 18 (e.g., tissue paper or a diaper backsheet). Such machines are well-known in the art. The rollers 10, 1 may be used, for example, in a method for manufacturing a diaper. The method, described below, is similar to that disclosed in U.S. Patent No. 5,147,347, with the additional step of embossing the backsheet of the diaper as described below. The disclosure of U.S. Patent No. 5,147,347 is hereby incorporated by reference.

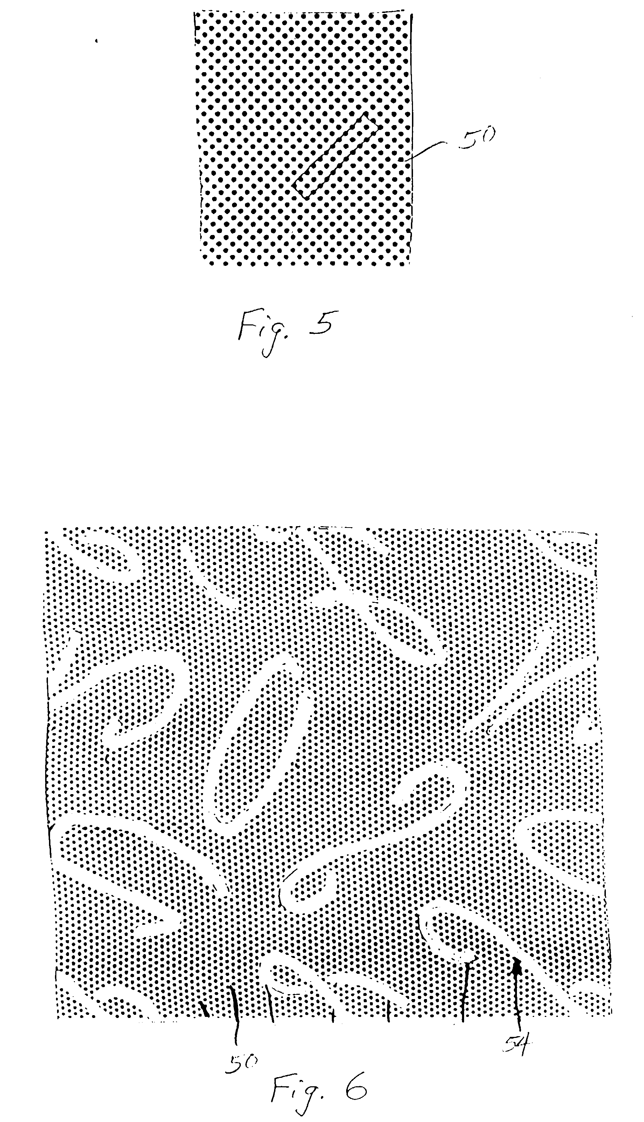

Fig. 4 illustrates a portion of a diaper 22 including a plurality of layers 26 and a backsheet 30. The backsheet 30 has been embossed by feeding the backsheet material between the rollers 10, 1 prior to assembling the diaper 22.

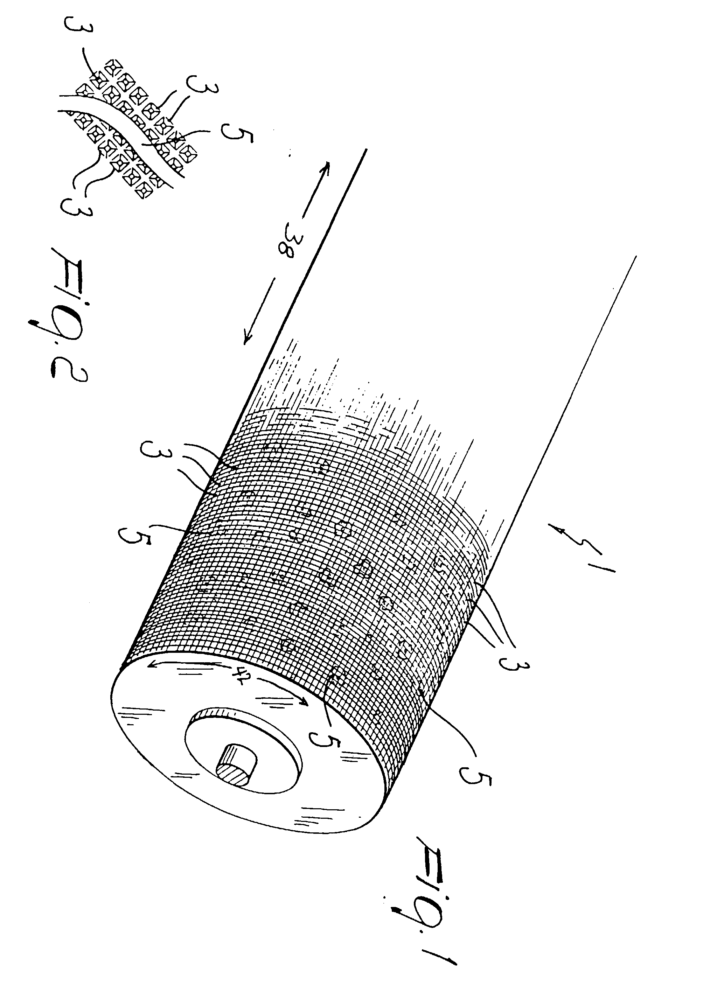

Referring now to Figs. 1 and 2, the hard roller 1 may be provided with a series of protrusions 3 arranged in parallel rows or another basic pattern. The protrusions 3 are formed on the hard roller 1 by machining or by a chemical process (e.g., etching). The forming step preferably includes a finishing step. Preferably, the protrusions 3 have the shape of truncated pyramids that extend radially outwardly from the outer surface of the hard roller 1. Each protrusion 3 is non-continuous in the cross-machine direction 38, i.e., from right to left in Fig. 1. In other words, the protrusions 3 do not extend all the way across the roller 1. The width of the protrusions 3 is measured in the cross-machine direction 38. A protrusion dimension of the protrusions 3 is measured in the circumferential direction 42. The width and protrusion dimensions are substantially less than the width and circumference, respectively, of the hard roller 1.

The hard roller 1 is produced by first forming a uniform series of protrusions 3, for example by machining, and then performing optional finishing operations. The next step is removing selected protrusions 3 or parts thereof from the surface of the hard roller 1 by, for example, a numeric-control machine (e.g., a milling machine), a knurling machine, or a laser engraving machine. This second step forms one or more ornamental patterns or designs 5 on the outer surface of the hard roller 1 among the remaining protrusions 3. The remaining protrusions 3 surround the patterns or designs 5.

Referring to Fig. 5, a matrix of dimples or spots 50 is embossed into the material 18 by the rows of protrusions 3 on the surface of the hard roller 1. A hard roller 1 having an unbroken array of protrusions 3 (e.g., one made by the above-described method using only the first step, and not the second step in which protrusions 3 are removed), will emboss a corresponding unbroken matrix or array of dimples 50 into the material 18.

A hard roller 1 having a pattern 5 formed among the protrusions 3 (e.g., one made by the above-described method using both the first and second steps), will emboss a broken matrix or array of dimples 50 into the material 18. Where there is no protrusion 3 on the hard roller 1, there will be no corresponding dimple 50 in the material 18. Thus a pattern or design is formed in the material 18.

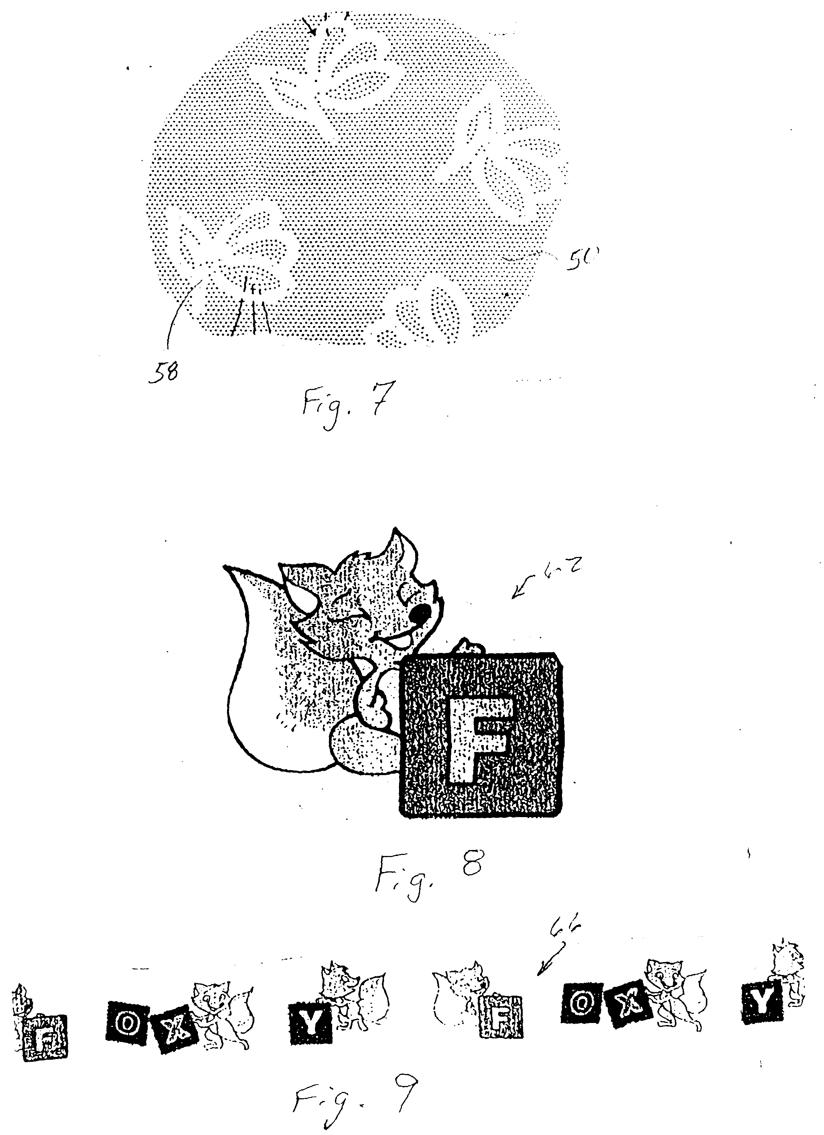

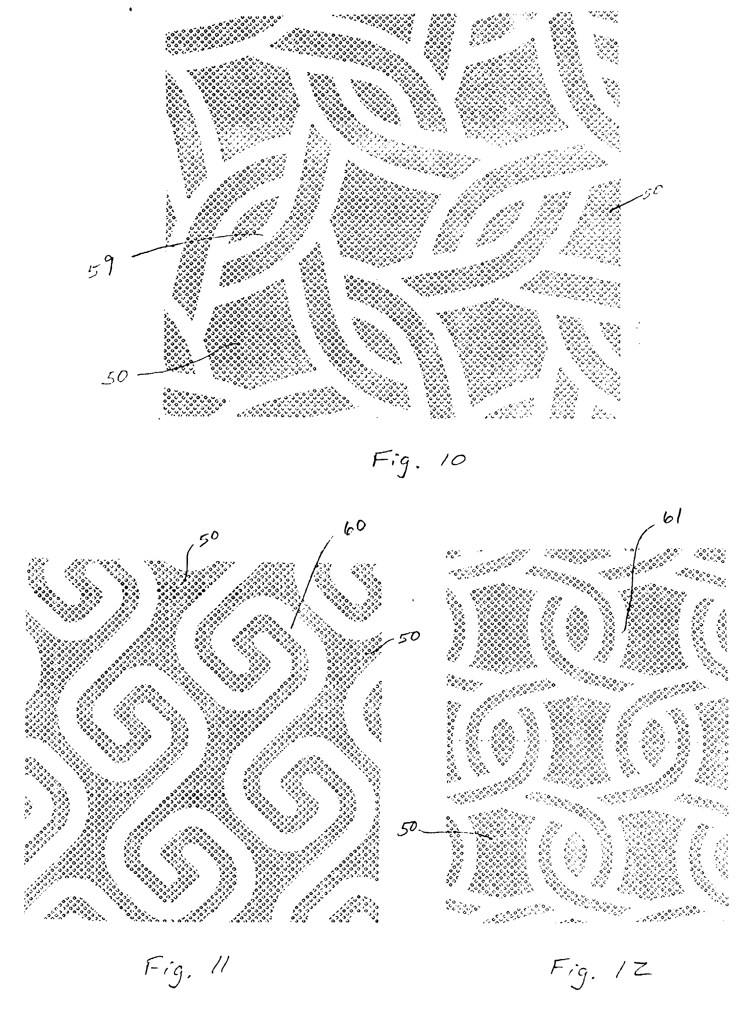

Figs. 6, 7, and 10-12 illustrate designs 54, 58, 59, 60, 61, respectively, in the material 18 created by removing selected protrusions 3 from the hard roller 1 and passing the material 18 between the hard and resilient rollers 1, 10. Figs. 8 and 9 illustrate alternative, more complex designs and corporate logos or brand names 62, 66 that are created with more complex arrangement of the protrusions 3 on the surface of the hard roller 1.

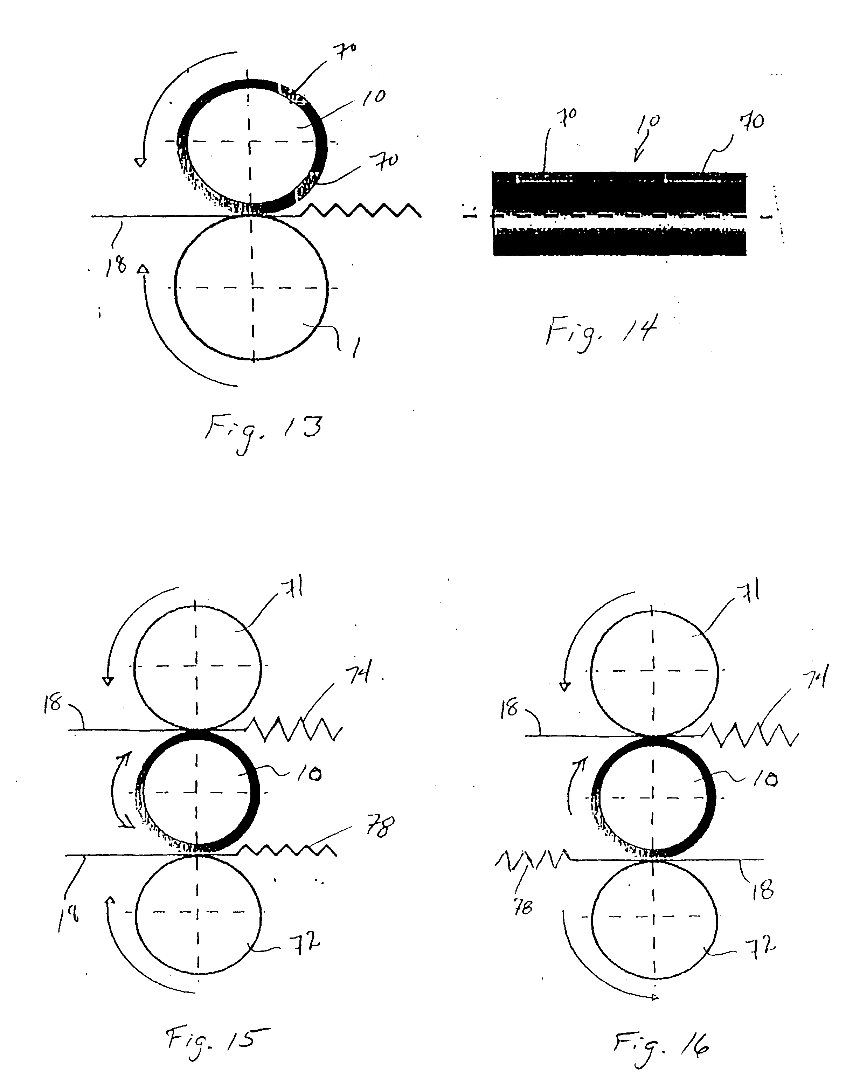

Figs. 13 and 14 illustrate an alternative set of rollers in which the resilient roller 10 has therein recesses 70. The hard roller 1 illustrated in Fig. 13 may include a solid array of protrusions 3, or may include a pattern 5 formed among the protrusions 3 by removing selected protrusions 3 or portions of protrusions 3. The recesses 70 in the resilient roller 10 will cause a pattern to be created in the material 18 when the material 18 is compressed between the resilient roller 10 and the hard roller 1. No dimples are embossed into the material 18 when a recess 70 is directly opposite the hard roller 1. This is so because the portion of the material 18 passing between the hard and resilient rollers 1, 10 is not compressed.

Fig. 15 illustrates another alternative set of rollers including a resilient roller 10 between first and second hard rollers 71, 72, respectively (also referred to as a "stacked" configuration). The roller 10 can rotate in either direction depending on which of the rollers 71, 72 is being employed at any given time. A sheet or web of material 18 can pass between the rollers 71 and 10, or another sheet or web 18 can pass between the rollers 72 and 10. The protrusions 3 on the hard rollers 71, 72 may be arranged in different patterns to create different embossing patterns on the material 18. For example, the roller 71 may be configured to create a simple design or solution 74 intended for all end users, and the roller 72 may be configured to create a custom design or solution 78 for a particular customer.

Fig. 16 illustrates an alternative stacked configuration, in which the resilient roller 10 rotates in one direction, and the first and second hard rollers 71, 72 are employed at the same time, and rotate in an opposite direction. In this configuration, the webs 18 are fed from opposite directions and the first and second solutions 74, 78 are created simultaneously.

Alternatively, a hard roller may be provided between first and second resilient rollers (not shown), and the resilient rollers may have different patterns of recesses to create different patterns on the sheets of material.

As an alternative to the illustrated embodiments, the method and apparatus may also be used to create two-ply tissue or other material. In that case, two sets of rollers, each including a resilient roller and a hard roller, are used to emboss a pattern on two different sheets of material. The two sheets of material, once embossed, are brought together, preferably between the hard rollers, and glued together with adhesive. Preferably, the protrusions of the hard rollers correspond to each other such that the respective sheets of material are bonded together under the pressure of essentially point contact between the protrusions of the respective hard rollers. Providing the design in a symmetrically identical manner on the two hard rollers also ensures the maximum number of gluing points between the two sheets or plies. The two sheets remain most stably associated where the largest possible number of protrusions of one sheet match those of another sheet.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A method of manufacturing a diaper, the method comprising the steps of:
(a) providing a sheet of diaper backsheet material;
(b) embossing the backsheet material by compressing the backsheet material between a hard roller and a resilient roller; and
(c) combining the embossed backsheet material with other materials to provide a finished diaper.

2. The method of claim 1, wherein step (b) improves the aesthetics of the material.

3. The method of claim 1, wherein step (b) includes making the sheet of material more cloth-like.

4. The method of claim 1, wherein the sheet of material includes relatively low-caliber raw materials having relatively low quality and low cost.

5. The method of claim 1, wherein step (b) includes embossing one of a brand name and graphics on the material.

6. The method of claim 1, wherein step (b) includes improving the hand feel of the material by making the material softer.

7. The method of claim 1, wherein the hard roller is made of steel.

8. The method of claim 7, further comprising the step of forming a recess in the resilient roller prior to compressing the sheet between the rollers.

9. A method for embossing a web of diaper backsheet material comprising, the method comprising the steps of:
(a) providing a first embossing roller by forming on the outer surface of a generally cylindrical roller a series of protrusions, and forming on the roller a pattern or design by at least partially removing a certain number of said protrusions so that the remaining protrusions surround the pattern or design;
(b) providing a second roller; and
(c) compressing the sheet of material between the first and second rollers.

10. A method for embossing a web of diaper backsheet material comprising, the method comprising the steps of:
(a) providing a first embossing roller having a width in the cross machine direction and having a circumference, by forming on the outer surface of a generally cylindrical roller a series of protrusions, each of said protrusions having a protrusion width in the cross machine direction, the protrusion width being substantially less than the width of the roller, and each of said protrusions having a protrusion dimension in the direction of the circumference of the roller, the protrusion dimension being substantially less than the circumference of the roller, and by forming on the roller a pattern or design by at least partially removing a certain number of said protrusions;
(b) providing a second roller; and
(c) compressing the sheet of material between the first and second rollers.
